# EUROPEAN PATENT APPLICATION

(11) **EP 1 795 143 A1**
(43) Date of publication of application: **13.06.2007**
(21) Application number: 06125349.8
(22) Date of filing: 04.12.2006
(51) Int. Cl.: A61B 19/00, A61B 10/00

(54) **Device for guiding needles, particularly biopsy needles or the like**

(30) Priority: 09.12.2005 IT SV20050040
(71) Applicant: Durante, Stefano, 40133 Bologna (IT); Rimondi, Eugenio, 40122 Bologna (IT)
(72) Inventor: Durante, Stefano, 40133 Bologna (IT); Rimondi, Eugenio, 40122 Bologna (IT)
(74) Representative: Karaghiosoff, Giorgio Alessandro

(57) **Abstract**

Device of the type described hereinbefore and that is a device for guiding needles, particularly biopsy needles, or the like, which device has mechanical means for supporting at least a needle, which mechanical means are borne by a frame provided with powered means for moving said needle at least according to one direction, preferably according to any spatial directions, means for generating a position reference between the spatial position of the needle and the patient position, or only of the operation area, that is only the operation anatomical district being further provided and wherein said position reference means are composed of the frame or part of the frame of an apparatus for acquiring inner diagnostic images which frame or part of the frame has a fixed and predetermined position and orientation with respect to the detected diagnostic image, that is to the scanning plane of the detected diagnostic image and the frame of said mechanical means supporting at least a needle or guide cannula being provided of movable means coupling the frame or part of the frame of the apparatus for acquiring diagnostic images which are such to determine a fixed and predetermined position and orientation relation of said supporting means and so of the needle and/or guide cannula with respect to the apparatus for acquiring diagnostic images and/or to the scanning plane of the image.

## Description

The invention refers to a device for guiding needles, particularly biopsy needles, or the like, which device has mechanical means for supporting at least a needle, which mechanical means are borne by a frame provided with powered means for moving said needle at least according to one direction, preferably according to any directions, means for generating a position reference between the spatial position of the neddle and the patient position, or only of the operation area, that is only the operation anatomical district being further provided.

At present there are various devices allowing to guide the physician during an operation for inserted a biopsy needle or the like, such as a thermoablation needle or a needle for making localized cryotheraphy operations or a needle for a localized administration of drugs.

Generally above needles are not directly inserted in the body, but cannulas for guiding needles are applied to the patient according to predetermined insertion paths that are intended to avoid interference with physiologically important anatomical parts in order to prevent health hazards for the patient. Thus the latter form ducts for inserting needles that are stable and prevent needles from interfering with tissues surrounding the neddle upon handling needles.

Thus, at present the needle insertion is preceded by the insertion of at least a guide cannula according to a predetermined direction that is studied and determined following indications about known surgical insertion routes.

Needle-guide cannulas can have even great diameters and the greatest problem for the surgeon when applying cannulas is the fact that, often the area or the organ or part thereof to be treated and reached by ends of the cannula can be reached only by passing with said cannula very close to vital importance organs or whose casual damage can lead to serious damages even to irreversible ones and sometimes also to patient death.

One of the most greatest problem in applying a needle or a needle-guide cannula is the fact that the physician cannot see which organs or parts are interfering with the needle or cannula path. When density or proximity, as well as the relative position of physiologically important organs is adverse, that is when there is an high concentration of such organs, then insertion of needle or needle-guide cannula is difficult and related to high risks. Considering the spinal anatomical district, then for example operations for inserting the needle or cannula have to occur under local anaesthetic, since the patient has to remain conscious in order to give feed-back indications that are important in order to check if needle or cannula are not damaging important nervous connections.

The fact of giving the physician means for making easier the guide of the needle removing at an high degree the manual guide of the needle and allowing also to see in real time or almost real time the inserting position of the needle and/or cannula therefore is a very important problem for increasing safety of such surgical applications.

At present systems for guiding the insertion of needles or cannulas are known attempting to give the physician the possibility to see the needle as well as the anatomical district surrounding it.

A part of these systems provides for example in real time or in a moment preceding the insertion of the needle or needle-guide cannula to make a three-dimensional scanning of the anatomical district wherein the neddle or cannula have to be inserted, generating an inner three-dimensional image of said area. Typical diagnostic imaging techniques allowing such scanning are radiographic technique, CT, magnetic resonance imaging and also ecographic imaging. The needle or cannula are provided with position and direction sensors and when making the insertion action they are monitored by means of visual image acquiring means (telecamera or the like) and diagnostic inner image acquiring means (ecographic or magnetic resonance imaging). Such two approaches, the three-dimensional diagnostic inner one acquired before the session inserting the needle and the one acquired during such session are then registered thanks to software and mathematical means and to the physician is given a virtual image of the inner part of the anatomical district upon which the image of at least the tip of the needle or guide cannula is superimposed during insertion.

These known devices or methods often use also movable or robotic arms or supports for the needle or cannula that are free as regards the position reference of the imaging apparatus, images regarding inner scanning and images regarding the outer visual approach, as well as the ones previously acquired being compared one with the other on the basis of common position references and so they are registered only by using mathematical and software means.

Though these systems can guarantee a result they are affected by considerable drawbacks. On one hand waste regarding the device and times is high. It is therefore necessary to acquire a three-dimensional image before the operation and so there is the need for imaging means of two types both visual outer one and inner one. Moreover registration of various images needs elaborate mathematical means and fast processors that often are subjected to malfunctions or so called crash. Registration between inner vision of the anatomical district and the detected position of the needle or cannula is however the result of a computation based on suppositions and on simplifications or on statistical objects and it can be still subjected to error. So there is no direct vision between the position of the needle or cannula and organs of the anatomical district wherein the insertion thereof occurs. Moreover the cannula or needle have to be provided with means detectable by sensor system allowing to detect the position and the orientation.

At present, without said systems, the monitoring of the needle or cannula position during insertion occurs at intervals by scanning the anatomical district wherein the needle or the guide cannula are inserted. Practically after a small step advancing the insertion of the needle or cannula by the physician the patient is taken in the scanning area of a diagnostic imaging apparatus, for example radiography, ecography, computerized tomography or magnetic resonance imaging, where one or more images of the anatomical district are acquired wherein the needle or cannula are inserted in order to control the position and direction thereof to be the correct one. After each step acquiring the control image/images, the patient is again taken out of the apparatus for a further advancing insertion step and so image acquiring, controlling and inserting steps follow one another till the needle or cannula have reached the operation area.

This procedure has also considerable drawbacks. Both the accuracy of controls regarding the needle or cannula position and regarding the subsequent insertion step and the increase in operation times are the greatest drawbacks causing the operation to become not only quite dangerous but also expensive.

The present invention aims to provide a device for guided insertion of needles or cannulas allowing to introduce a needle or a cannula in an anatomical district of a patient in a simple, fast and quite inexpensive way, an accurate and direct visual control of the needle or cannula position being at disposal with respect to organs in the anatomical district which is almost at real time and it does not require statistical or mathematical means as well as expensive devices and moreover it allows a reduction of the dose absorbed by the patient.

The invention achieves above aims by providing a device of the type described hereinbefore and that is a device for guiding needles, particularly biopsy needles, or the like, which device has mechanical means for supporting at least a needle, which mechanical means are borne by a frame provided with powered means for moving said needle at least according to one direction, preferably according to any spatial directions, means for generating a position reference between the spatial position of the needle and the patient position, or only of the operation area, that is only the operation anatomical district being further provided and wherein said position reference means are composed of the frame or part of the frame of an apparatus for acquiring inner diagnostic images which frame or part of the frame has a fixed and predetermined position and orientation with respect to the detected diagnostic image, that is to the scanning plane of the detected diagnostic image and the frame of said mechanical means supporting at least a needle or guide cannula being provided of movable means coupling the frame or part of the frame of the apparatus for acquiring diagnostic images which are such to determine a fixed and predetermined position and orientation relation of said supporting means and so of the needle and/or guide cannula with respect to the apparatus for acquiring diagnostic images and/or to the scanning plane of the image.

Particularly, in the present needle-guide device, the needle is always held by the mechanical support means within the scan plane of the acquired diagnostic image so that a fixed relationship exists between the position and orientation of the needle and the scan plane. As a result, when the scan plane changes, the mechanical needle-supporting means will move the instrument to a new position.

Therefore the invention is based on the fact that each apparatus for acquiring diagnostic images has a fixed and definite reference or a fixed and predetermined position and orientation relation of the image plane with respect to at least a part of the frame of the apparatus. So by fastening the supporting frame of a needle or guide cannula to said frame part of the apparatus acquiring diagnostic images said supporting frame has a position and orientation that can be defined in an accurate and univocal way with respect to the scanning plane of the image and so to the needle or guide cannula borne by said frame.

Thanks to this arrangement, the invention allows to obtain the automatic or servo-assisted guide of the needle or cannula during insertion and direct visual control of the position and orientation of the needle or cannula with respect to the anatomical district wherein it is inserted.

The frame supporting the needle or guide cannula can be of any type and particularly it can be as a supporting arm, with an head supporting the needle or guide cannula and which arm is movable by powered means according to one or more directions to a predetermined and monitorable or adjustable extent, remote control means being provided for motors moving the arm.

That allows for example to keep the patient inside the apparatus for acquiring diagnostic images even during insertion steps. So the image of the patient of the operation area wherein the needle or guide cannula is inserted can be acquired at all times thus allowing to see directly and at real time how the needle and cannula moves inside the patient body.

Position and orientation references between the needle or cannula and the detected image being fixed and defined by position and orientation relations between the apparatus frame and so means supporting the needle thereto and the scanning plane of the image, the image for controlling the position of the needle and the anatomical district wherein it is inserted are surely free from errors and so are accurate and reliable.

According to a further advantageous feature, in addition to the manual control, that can occur by control means like levers or joystick, the position and orientation and advancing of the needle in insertion state can take place automatically on the basis of a path defined by the physician by indicating the desired position of the needle or cannula tip for example by means for pointing or indicating the position on the image displayed on a screen.

To this aim, the device according to the invention for example can provide:
an image computing unit transforming scanning signals along at least a scanning plane into image data;
a monitor displaying the image in grey-scale and/or in colours of image data;
a pointing device like a mouse or the like connected to the computing unit and whose position with respect to the displayed image is represented by a pointing graphic member;
means for entering the control advancing the needle with reference to the position indicated by the pointer on the image displayed on the monitor;
means for determining control signals for activating powered means for moving and/or orienting the needle-holder head on the movable supporting frame, which means determine control signals on the basis of fixed position and orientation references between the scanning plane of the image and the frame of the apparatus and so of means supporting the needle and the needle itself,
all that in such a way that the point of the image indicated by the pointer is determined as the moving point of the tip of the needle or cannula in the subsequent insertion step after the advancing step has been entered, position data being determined on the basis of the position of the image point indicated by the pointer with reference to the preceding position of the tip of the needle or cannula and to the imge of the anatomical district transformed in data modifying the position and orientation of the needle by the corresponding activation of powered means moving the frame supporting the needle or cannula.

According to a further feature, means for controlling powered means moving the frame supporting the needle or cannula can be composed of a pointer of the so called joystick type a switch being further provided for connecting the joystick as a direct control mean or as an indirect control mean and that is it moves a pointing image on the image detected of the anatomical district, control signals being determined by position relations of pointing image with the image of the anatomical district.

In the case of a direct control of movement of means supporting the needle by the joystick or other type of levers it is possible to provide reduction gear means between the movement of the joystick or levers and the corresponding movement of means supporting the needle. Said reduction gear means can be such that to a movement of the joystick corresponds a movement for a smaller travel of supporting means.

Moreover reduction gear means can be of the electronic type and that is they act on the control signal produced by the joystick there being also possible to provide changers of the gearing down ratio that can be controlled or adjusted by the user.

A particularly advantageous embodiment provides the needle or needle-guide cannula to be borne by a supporting frame fastened to the frame of an apparatus for acquiring diagnostic images and which apparatus makes the imaging at least according to a predetermined scanning plane, while the needle or needle guide are borne by said frame with their axis in a position parallel or coinciding with said scanning plane.

In this case, the frame supporting the needle or the needle-guide cannula, has a needle-holder or cannula-holder head having means for translating the needle or cannula in the direction of the longitudinal axis thereof and inside said scanning plane or to a plane parallel thereto.

Still according to said advantageous embodiment variant, the frame of the apparatus acquiring images can be moved such that the scanning plane along which an image is acquired is moved in various orientation conditions, the frame supporting the needle or the guide cannula being fixed to said movable frame of the apparatus so that the needle or cannula are moved in space in a way corresponding to the movement of the scanning plane.

A preferred embodiment provides the frame of the apparatus acquiring images to be angularly movable about an axis parallel or included in the scanning plane, said scanning plane being angularly rotated with respect to said axis and the needle or guide cannula being also rotated together with said scanning plane and at the same angular extent, so that the axis of the needle or neddle-guide cannula mantein the parallel relation or said axis remains included inside the scanning plane for any orientation thereof.

Moreover a further improvement provides the needle or needle-guide cannula to be supported in an adjustable way according to any directions in the scanning plane or in a plane parallel to said scanning plane.

From a manufacturing point of view, this can be obtained by providing a curved sliding guide, preferably a circular one and integral with the frame of the apparatus for acquiring images and along which a saddle or slide bearing means supporting the needle and/or guide cannula is mounted that can be moved and fixed in place manually or by powered means.

Said embodiment of the invention has a particular advantageous application in combination with an apparatus for acquiring diagnostic images, of radiological type, having means for scanning the image along a scanning plane housed in an annular frame, whose inner space is the space for housing the patient the image scanning plane being composed of the median plane subtended by said annular frame, while said annular frame can be tilted about a diametral axis means supporting the needle or the needle guide cannula being composed of an arm fastened to an end of said annular frame and which opposite end extends in the area of the scanning plane and it bears a needle-holder head wherein the needle or cannula are retained in a position coinciding with said scanning plane or with a plane parallel thereto and while the annular frame has a coaxial or substantially coaxial guide that is an annular one or like an annular sector for a saddle or slide moving means fastening the end of the arm supporting the needle.

Still according to a further advantageous embodiment of the invention, the needle-holder head is made like a four bar polygon or better like a four bar linkage, wherein the needle or the needle-guide cannula are retained in a position coinciding with a diagonal of said four bar linkage, such that the activation of the four bar linkage determines the to and fro movement for a predetermined travel of the needle or needle-guide cannula in a direction parallel to itself and its longitudinal axis.

A variant provides the needle-holder head to be made like a pantograph, the needle or cannula being borne by a member provided at a pivot point between two links or arms of said pantograph and the pantograph being made such that said pivot point moves along a straight path when opening and closing the pantograph.

This embodiment is particularly advantageous, since the frame supporting the needle is quite simple and so inexpensive and sure. By keeping the needle in a position coinciding with the scanning plane makes the needle completely visible at all times in the acquired image and at all times oriented parallely to the plane along which the image is acquired. This avoids offset distance problems due to perspective effects that can take place if the needle or cannula are not retained at least in a parallel position or better in a position such to be included in the scanning plane.

So the surgeon using the device clearly understands the situation without the need to use complicated and very sensitive and not strong techniques combining real views with previously acquired views and inner views with outer views, trying to generate position references between different worlds of inner images and outer images.

The invention provides further improvements that are object of subclaims.

Features of the invention and advantages deriving therefrom, will be clear from the following description of a not limiting embodiment shown in annexed drawings, wherein:

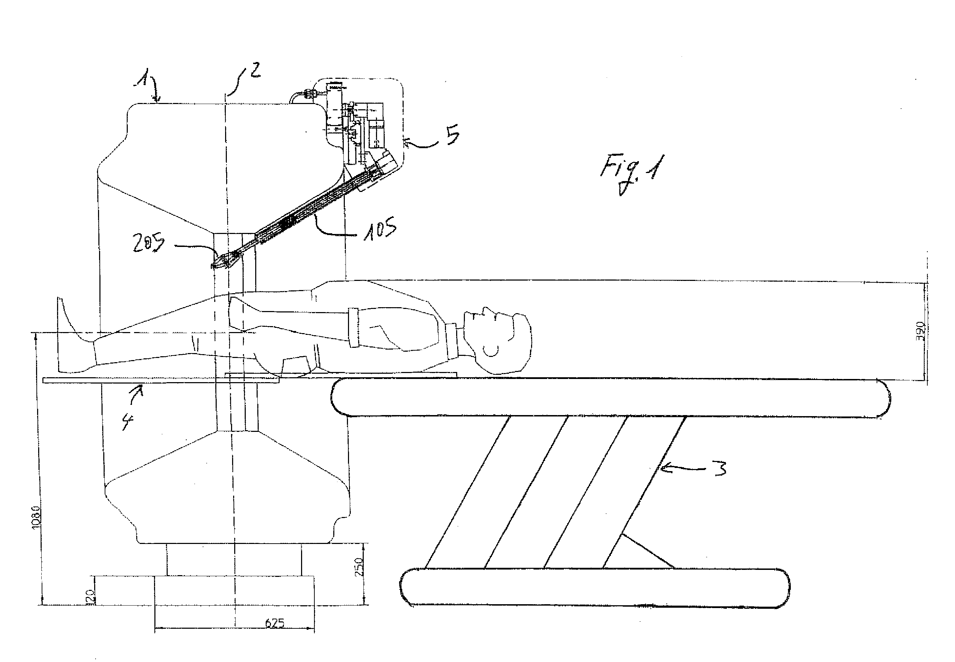

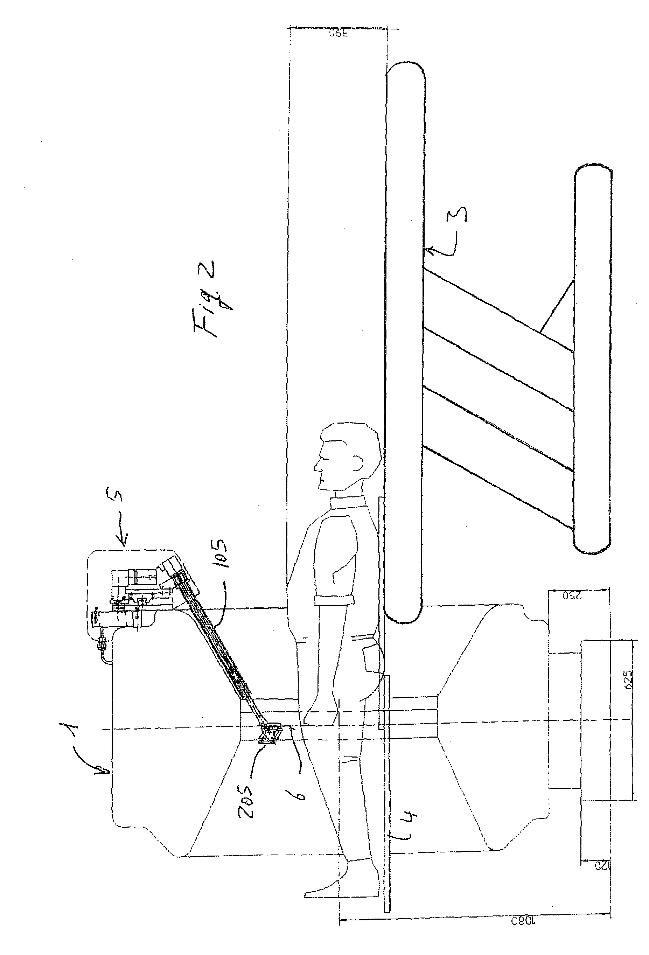

Figures 1 and 2 are a device according to the present invention in combination with a preferred embodiment of an apparatus for acquiring diagnostic images of the type at present the more frequently used for monitoring operations introducing biopsy needles or the like or guide cannulas for said needles, in figure 1 the needle being in the retracted position and in figure 2 the needle being in advanced introducing position respectively.

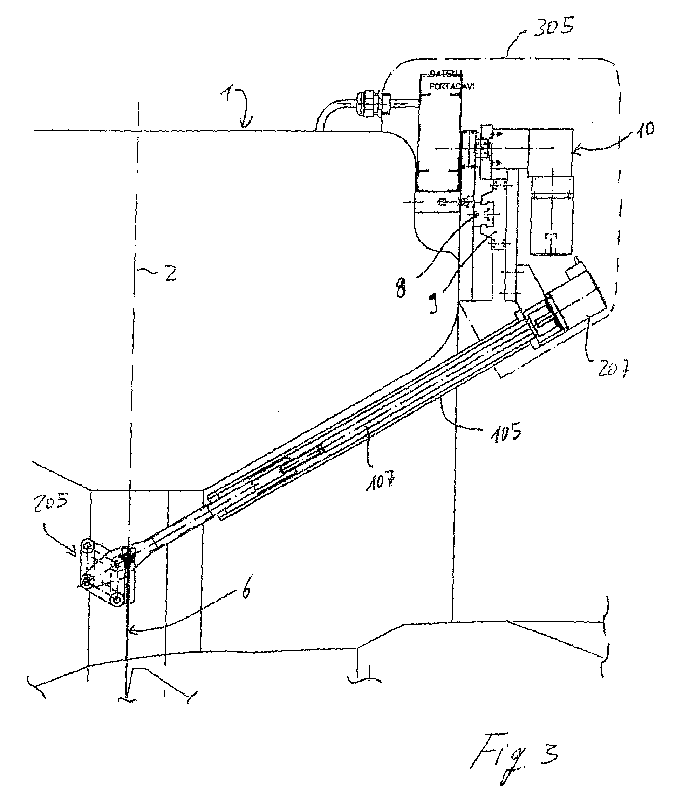

Figure 3 is an enlarged detail regarding means for supporting the needle or the guiding needle cannula.

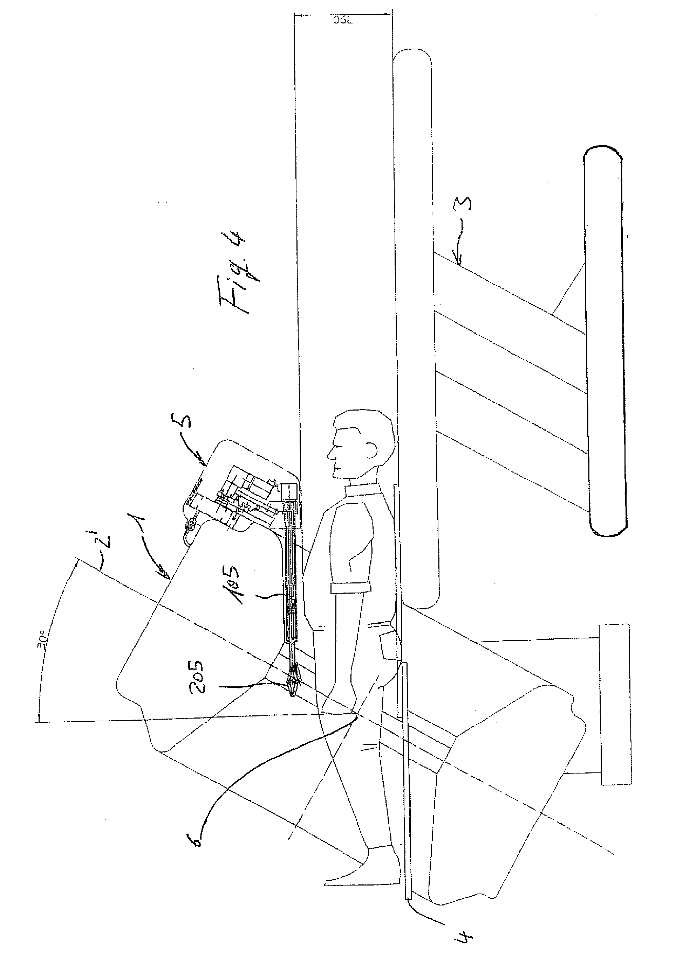

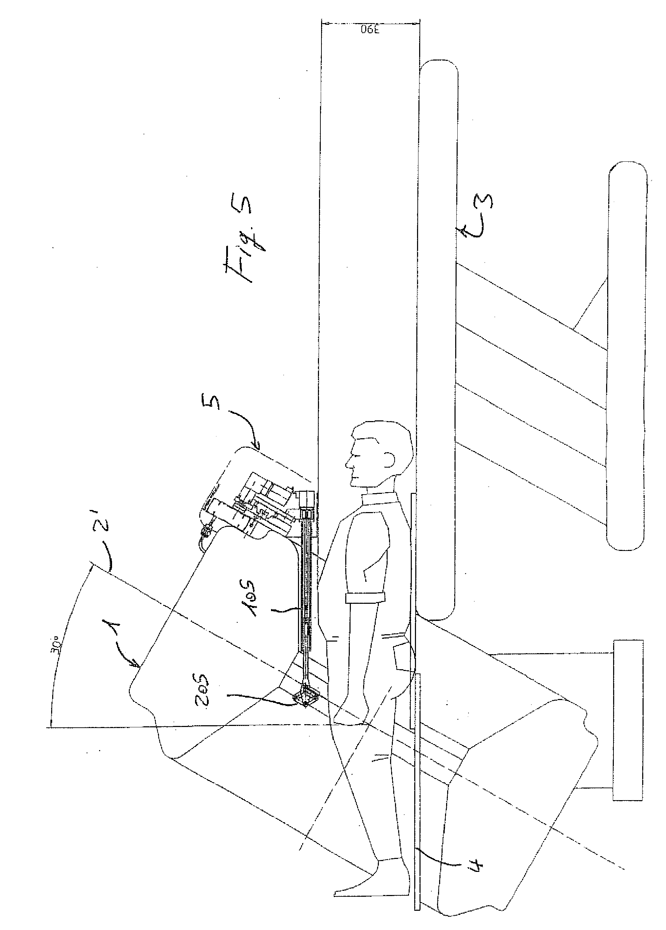

Figs. 4 and 5 are views similar to figures 1 and 2, wherein nerverthless the frame of the apparatus for acquiring images bearing means for acquiring images along an annular element and the corresponding scanning plane as well as means supporting the needle or needle-guide cannula are oriented as tilted at 30° with respect to the position in figure 1 and 2.

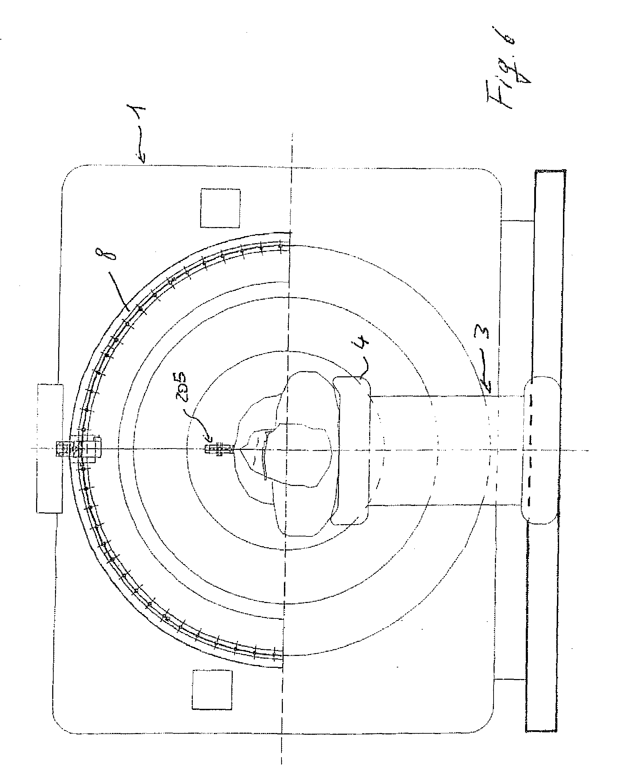

Figure 6 is a view in the direction of the axis of the annular frame of the apparatus for acquiring images showing the curved guide for sliding the saddle bearing the frame supporting the needle or needle-guide cannula.

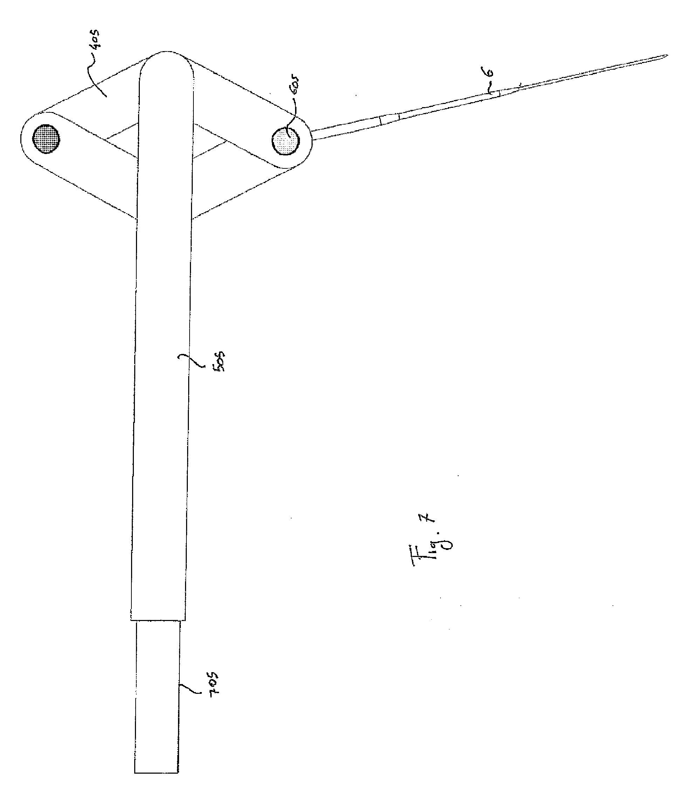

Figures 7 and 8 and 9 and 10 are schematic and side views and manufacturing and perspective views respectively of a needle-holder head like a pantograph or a four bar linkage, in closed and retracted position of the needle and in open and advanced position in the insertion direction of the needle respectively.

Referring to figures, though they show a preferred and particularly simple, inexpensive and effective combination of the device according to the present invention with an apparatus for acquiring images, it is to be noted that such preferred combination is to be intended not limitative of the concept and generic inventive teaching mentioned above and claimed below, but it is one of many examples of it.

Said concept or said generic teaching for supporting insertion needle or cannula consists in using frame parts of an apparatus for acquiring images which have a fixed, definite and known spatial position and orientation with respect to the plane along which the image is acquired by the apparatus, the needle or needle-guide cannula being retained in predetermined positions and with predetermined orientations with respect to said scanning plane, such to have at all times a position and orientation relation of the needle corresponding to a greatest extent to the real one with reference to organs visible in the image.

Obviously when the needle is kept in the scanning plane, that is with its axis included in said plane, distances deducible from the image are the most exact ones and the least distorted ones with respect to reality.

For this reason the embodiment that will be described in the following with aid of drawings has to be considered as the preferred one, since it allows from the image to have a rappresentation of the position of the needle in the examination area that is the most exact and the least distorted with a minimum burden regarding computational hardware and software and so it allows to remove device parts that are subjected to breaking or malfunctions.

Particularly, in the present device the needle is always held by the mechanical support means within the scan plane of the acquired diagnostic image so that a fixed relationship exists between the position and orientation of the needle and the scan plane. As a result, when the scan plane changes, the mechanical needle-supporting means will move the instrument to a new position.

The apparatus for acquiring images shown in figures is an apparatus for acquiring radiographic images and particularly tomographic images and it comprises an imaging scanner composed of an annular door 1. This door determines a cavity and a plane along which the image is acquired, so called scanning plane that is denoted by 2 and by the broken line in figures.

The scanning plane has always a repeatable and predetermined position with respect to the frame of the annular door 1.

The patient is passed through the opening of the annular door 1 by a supporting examination table 4 that is slidable mounted upon a base 3. The supporting examination table 4 is inserted with its longitudinal axis parallel to the axis of the annular door 1 being carried overhangly by the base 3.

When the examination table slides through the opening of the annular door 1, the patient is crossed by the scanning plane. The acquisition of an image in sequence along the scanning plane allows to make a set of section images of the patient in various body areas or points after each image the examination table being moved by a predetermined advancing step and so it is possible to generate three-dimensional images formed by the sequence of said section images acquired along the scanning plane 2.

To the frame of the annular door 1 there is fastened an arm 105 of a supporting frame 5 of a needle or needle-guide cannula to be inserted. The needle or the needle-guide cannula indicated by 6, is mounted in a needle-holder head 205 that is provided at the end of said arm.

The needle-holder head 205 is made such that the needle or the needle-guide cannula 6 is in position with its longitudinal axis parallel to or included in the scanning plane 2 defined by the annular door 1 of the image acquiring apparatus.

The head 205 comprises means for moving the needle or the cannula 6 in a direction parallel to their longitudinal axis, that is the insertion direction, which means are powered means and are driven by driving means 107 of the drive motion provided by the motor 207. Driving means 107, for example a driving shaft are housed and passed through the arm 105 that is suitably made as an hollow or tubolar one. The motor 207 can be of any type and particularly it is a pneumatic or hydraulic motor in order to avoid means electrically disturbing the imaging scanner.

The motor 207 is housed in a box 305 that on the outside closes the end fastening the arm 107 to the frame of the annular door 1 and covering also fastening means.

Referring particularly to figures 3 to 6, the end of the arm 105 opposite to the needle-holder head 205 and more distant from the scanning plane and possibly on the outside of the annular door 1, is fastened thereto at least by a guide 8 for a slider or saddle 9 that is slidable along said guide 8 both by manually operation and by means of a powered member 10.

Particularly as in figure 6 said guide is a curved guide, preferably like a circle section coaxial to the central axis of the opening defined by the annular door 1. The guide 8 is fastened to an head side of said frame of the annuler door 1, while the saddle 9 bears the end of the arm 105 opposite to the one provided with the needle-holder head 205. Due to this arrangement, the needle or the needle-guide cannula 6 can be oriented according to any angular directions in the plane determined by the scanning plane 2 or in a plane parallel thereto.

According to a possible variant, instead of providing the arm 105 directly fastened to the saddle 9, the latter can in turn bears a radial guide (not shown) upon which a slidable slider is mounted on said radial guide and to which the arm 105 is fastened. So if necessary all that allows to possibly adjust the radial position of the arm for roughly presetting the needle-holder head in a condition approaching the same and so the needle to the insertion site in the body to be treated. The further movement of the needle or cannula 6 occurs thanks to means of needle-holder head 205 that as it will be described in details below it can be made in such a way to allow a more accurate micrometric movement with which the needle or cannula 6 is moved along the insertion path till the final desired position.

A possible further variant also not shown but easy to make consists in providing in case the arm to be adjustable in length such to allow a movement of the needle in eccentric position with respect to the scanning plane, particularly in a plane parallel to the scanning plane 2 and laterally offset with respect to said scanning plane. Even in this case possible motors can be housed in the box 305.

Though the needle-holder head 205 can be possibly mounted on the arm 105 in an tilting way about an axis perpendicular to the needle and parallel to the scanning plane, in order to give the axis of the needle a tilting with respect to the scanning plane 2 along a plane incident with said scanning plane 2, in the preferred embodiment, to this aim the invention provides to use the fact that generally apparatus for acquiring images allows the tilting of the scanning plane with respect to the patient body particularly due to the oscillation of the frame of the annular door 1 about an axis parallel or included in the scanning plane and perpendicular to the axis of the examination table or of the insertion direction of the patient or to the opening defined by said frame of the annular door 1.

This arrangement allows to avoid making the needle-holder head 205 and means for driving and moving the needle 6 even more complicated. By keeping rigidly constrained the arm 105 to the frame of the annular door 1 with reference to said tilting, the frame 5 supporting the needle or the needle-guide cannula 6 and the needle-holder head 205 as well as the needle or cannula 6 are tilted together with the scanning plane 2, keeping the position parallel to or coinciding with said scanning plane for any inclination of the scanning plane.

Thus when the image necessary for properly controlling the insertion position of the needle has to occur according to a scanning plane having a predetermined orientation, the needle is automatically kept in the position parallel to the image plane and so perspective offsets of the needle position with respect to organs or to anatomical structures of the image are avoided. All that can be seen from the image therefore corresponds perfectly to reality. The choice of the present preferred embodiment allows to mantain the needle-holder head 205 very simple, inexpensive and sure guaranteeing to the greatest extent the correspondence between the situation deducible from the image and the real one.

This condition is shown in figures 4 and 5. In these figures, the only difference with respect to figures 1 and 2 is the fact that the frame of the annular door 1 is tilted at 30° with respect to an axis perpendicular to the plane of the sheet and passing at point O. As it can be clearly seen in figures, the needle-holder head 205 is at the same position as in figures 1 and 2 both regarding the retracted position of the needle 6 and the maximum advancing position of the needle 6 in insertion direction and the axis of the needle or cannula 6 is still parallel to or included in the scanning plane even if the latter is tilted and it is denoted by 2'.

Even as regards the needle-holder head 205, different embodiments are possible. In this case mechanical conditions to be satisfied by such head are two. The head must be such to move the needle parallely to itself, that is mantaining at all times the needle axis coinciding with the straight line ideally extending said axis and moreover it has to allow micrometric movements, guaranteeing at the same time a force sufficient for the penetration.

In addition to this, the needle-holder head must have a simple manufacture and such to be easily subjected to cleaning and sterilization procedures.

In the preferred embodiment, the needle-holder head 205 is made like a four bar polygon and particularly like a four bar linkage or like a pantograph.

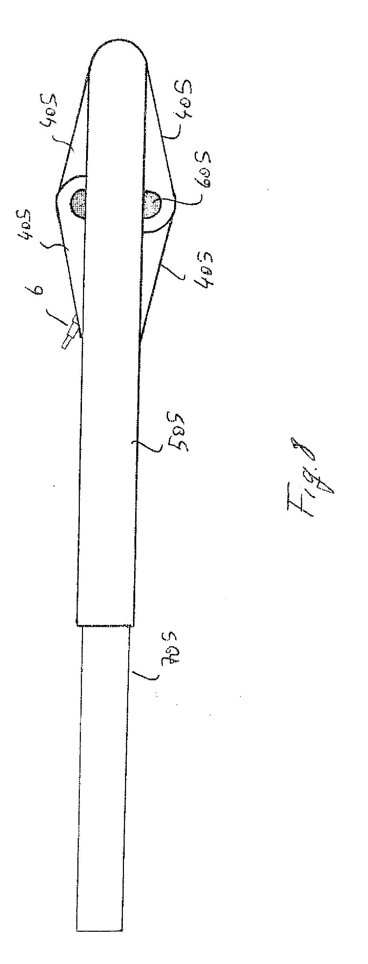

Four bars 405 are joint one with the other two pivot points being stably constrained along a same axis 505. One of these pivot points is provided at the free end of a rod 505, while the other pivot point is provided joint to said rod 505 but axially slidable along it, as can be seen in figures 7 and 8. The needle 6 is borne by a member in the area of one of pivots 605 not coinciding with rod 505. By widening and flattening the linkage advancing and retracting movement of the needle 6 or guide cannula is obtained. The widening and shortening control action may occur in a very simple way by using pushing or pulling means (not shown) for example a rod that can slide inside the rod 505 and acting on the slidable pivot point in the axial direction of the rod 505.

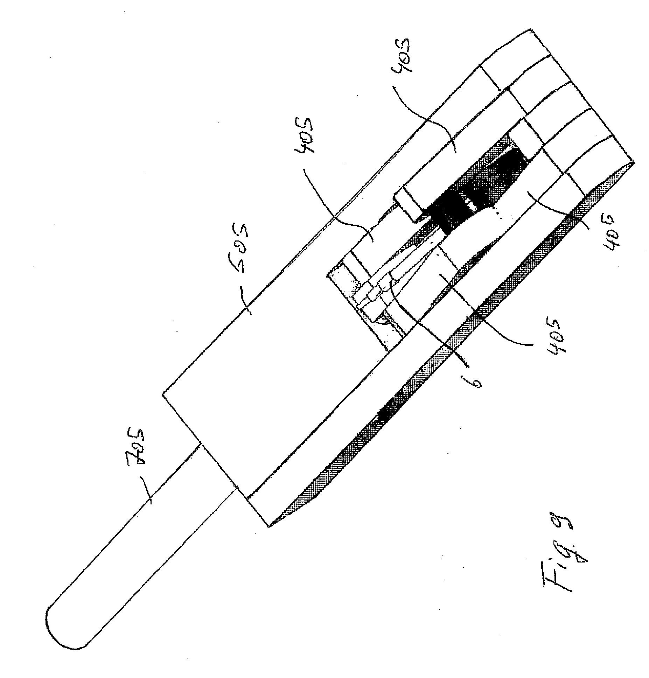

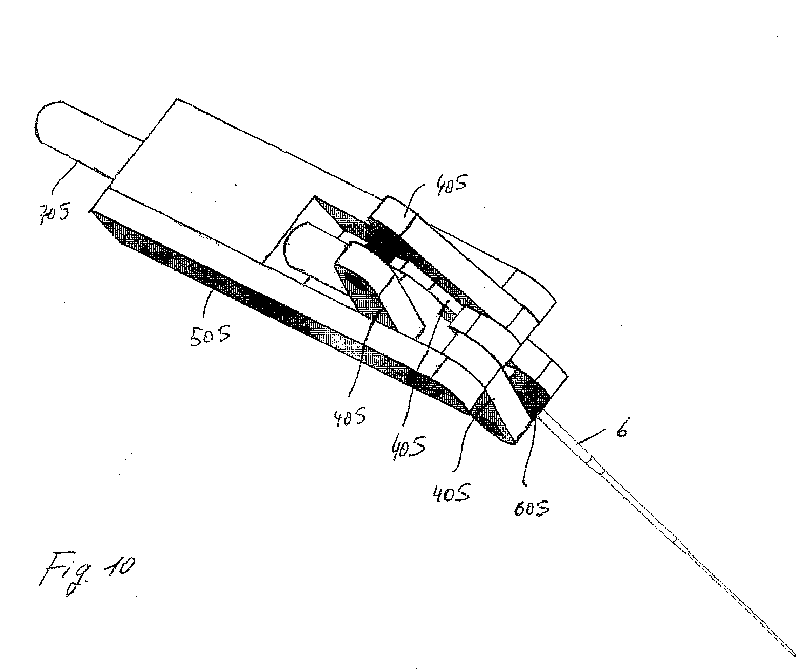

Perspective views of figures 9 and 10 show in more detail the rod 505, the control slidable rod 705, bars 405, the pivot point 605 bearing the needle 6 or cannula and two pivot points coinciding with the rod 505, that is the end one and slidable one controlled by the control rod 705.

As already said above, the described and shown embodiment is only one of the possible embodiments. Besides the mechanical control it is possible for example to provide the needle to be mounted at the end of a pneumatic, hydraulic or mechanical cylinder that is operated in two directions extending outwards and returing the stem, achieving the same task of the pantograph. Other types of pantograph are also possible.

Still according to a possible variant, it is possible to provide on the same frame of the apparatus for acquiring images to be mounted various frames supporting needles or guide cannulas in order to provide the contemporanous insertion of various needles or cannulas when this is required or necessary. That is possible also because the frame supporting the needle or needle-guide cannula is quite small and so it is possible to provide various supporting frames positioned in different points of the frame of the annular door 1 and particularly of the circle-sector like guide 8.

Still another variant can provide that in combination with the apparatus for acquiring images to which frame the frame for supporting the needle or cannula is fastened there is provided a further device for acquiring images for example in the shown case an apparatus for detecting ecographic images, whose probe is borne in the proper acquiring position both manually and by means of a further supporting frame constrained or that can be fastened to the frame of the door or by means of the frame supporting the needle itself.

Still according to a further variant embodiment, the frame supporting the needle or cannula may be of the type that can be assembled or disassembled from the apparatus for acquiring images.

As regards control of tasks of needle-holder head and possible movements of the frame supporting the needle, these can be manual control means, like mouse, joystick or control levers controlling motors operating movements provided according to variants of the invention that are implemented in a device. Advantageously it is possible to provide a reduction that can be also adjustable providing a relation between the travel of control means and the travel of supporting means during movement such to make easier or to aid the control. For example by acting on the control signal generated by a control lever, the latter may have a quite big travel that on the contrary produces micrometric movements or fractions of movements of the control lever. It is also possible to provide the gearing down ratio to be adjustable by suitable adjusting means.

The movement of the needle or cannula in the insertion direction and so the movement of the frame supporting this needle or this needle-guide cannula can be controlled also by a simple graphic interface GUI that is graphic user interface. In this case by a pointer on a screen displaying the operation area the position of the needle desired to be reached in an insertion step to performed is defined or indicated. Once the pointer is in said position on the image an agreement signal is entered. A program computes the advancing of the needle with reference both to the real actual position and in the image and it computes the advancing step to be performed by the needle for reaching said position. Before actually carrying on the advancing step of the needle the device can make an advancing virtual step that is shown to the user as a short sequence of frames and wherein the image of the needle or cannula are virtually moved on the basis of data obtained by computation. If the movement made in mode produces interfering situations of the needle with areas or organs with which that has to be avoided, the device can emit a warning and suggests to check the situation and in case to redefine the advancing step, by indicating a new desidered advancing position.

Said tasks may be carried on by suitable softwares that can be loaded and performed by processing means that generally are already provided in modern apparatus for acquiring diagnostic images.

## Claims

1. Device of the type described hereinbefore and that is a device for guiding needles, particularly biopsy needles, or the like, which device has mechanical means for supporting at least a needle, which mechanical means are borne by a frame provided with powered means for moving said needle at least according to one direction, preferably according to any spatial directions, means for generating a position reference between the spatial position of the needle and the patient position, or only of the operation area, that is only the operation anatomical district being further provided and wherein said position reference means are composed of the frame or part of the frame of an apparatus for acquiring inner diagnostic images which frame or part of the frame has a fixed and predetermined position and orientation with respect to the detected diagnostic image, that is to the scanning plane of the detected diagnostic image and the frame of said mechanical means supporting at least a needle or guide cannula being provided of movable means coupling the frame or part of the frame of the apparatus for acquiring diagnostic images which are such to determine a fixed and predetermined position and orientation relation of said supporting means and so of the needle and/or guide cannula with respect to the apparatus for acquiring diagnostic images and/or to the scanning plane of the image.

2. Device accordino to claim 1, **characterized in that** the apparatus for acquiring diagnostic images has a fixed and definite reference or a fixed and predetermined position and orientation relation of the image plane that is scanning plane with respect to at least a part of the frame of the apparatus, while the frame supporting a needle or a guide cannula is fastened to said frame part of the apparatus acquiring diagnostic images ahd it has a position and orientation that can be defined in an accurate and univocal way with respect to the scanning plane of the image and so to the needle or guide cannula borne by said frame.

3. Device according to claims 1 or 2, **characterized in that** it has automatic or servo-assisted guide means for the needle or cannula during insertion and direct visual control means for the position and orientation of the needle or cannula with respect to the anatomical district wherein it is inserted.

4. Device according to one or more of the preceding claims, **characterized in that** the frame supporting the needle or guide cannula can be of any type and particularly it can be as a supporting arm, with an head supporting the needle or guide cannula and which arm is movable by powered means according to one or more directions to a predetermined and monitorable or adjustable extent, remote control means being provided for motors moving the arm.

5. Device according to one or more of the preceding claims, **characterized in that** the patient is placet inside the apparatus for acquiring diagnostic images even during steps inserting the needle or cannula, the image of the patient of the operation area wherein the needle or guide cannula is inserted being acquired directly and at real time, position and orientation references between the needle or cannula and the detected image being fixed and defined by position and orientation relations between the apparatus frame and so means supporting the needle fastened thereto and the scanning plane of the image.

6. Device according to one or more of the preceding claims, **characterized in that** the movement inserting the needle or cannula is made by a control by means of control means like levers or joystick.

7. Device according to one or more of the preceding claims, **characterized in that** the movement inserting the needle or cannula and the position and orientation and advancing of the needle in insertion state takes place automatically on the basis of a path defined by the physician by indicating the desired position of the needle or cannula tip by means for pointing or indicating the position on the image displayed on a screen.

8. Device according to claim 7, **characterized in that** it comprises:
an image computing unit transforming scanning signals along at least a scanning plane into image data;
a monitor displaying the image in grey-scale and/or in colours of image data;
a pointing device like a mouse or the like connected to the computing unit and whose position with respect to the displayed image is represented by a pointing graphic member;
means for entering the control advancing the needle with reference to the position indicated by the pointer on the image displayed on the monitor;
means for determining control signals for activating powered means for moving and/or orienting the needle-holder head on the movable supporting frame, which means determine control signals on the basis of fixed position and orientation references between the scanning plane of the image and the frame of the apparatus and so of means supporting the needle and the needle itself,
all that in such a way that the point of the image indicated by the pointer is determined as the moving point of the tip of the needle or cannula in the subsequent insertion step after the advancing step has been entered, position data being determined on the basis of the position of the image point indicated by the pointer with reference to the preceding position of the tip of the needle or cannula and to the imge of the anatomical district transformed in data modifying the position and orientation of the needle by the corresponding activation of powered means moving the frame supporting the needle or cannula.

9. Device according to one or more of the preceding claims, **characterized in that** means for controlling powered means moving the frame supporting the needle or cannula can be composed of a pointer of the so called joystick type a switch being further provided for connecting the joystick as a direct control mean or as an indirect control mean and that is it moves a pointing image on the image detected of the anatomical district, control signals being determined by position relations of pointing image with the image of the anatomical district.

10. Device according to one or more of the preceding claims, **characterized in that** in the case of a control of movement of means supporting the needle by the joystick or other type of levers it is possible to provide reduction gear means between the movement of the joystick or levers and the corresponding movement of means supporting the needle and wherein said reduction gear means can be such that to a movement of the joystick corresponds a movement for a smaller travel of supporting means.

11. Device according to claim 10, **characterized in that** reduction gear means can be of the electronic type and that is they act on the control signal produced by the joystick there being also possible to provide changers of the gearing down ratio that can be controlled or adjusted by the user.

12. Device according to one or more of the preceding claims, **characterized in that** the needle or needle-guide cannula are borne by a supporting frame fastened to the frame of an apparatus for acquiring diagnostic images and which apparatus makes the imaging at least according to a predetermined scanning plane, while the needle or needle guide are borne by said frame with their axis in a position parallel or coinciding with said scanning plane.

13. Device according to one or more of the preceding claims, **characterized in that** the frame supporting the needle or the needle-guide cannula, has a needle-holder or cannula-holder head having means for translating the needle or cannula in the direction of the longitudinal axis thereof and inside said scanning plane or to a plane parallel thereto.

14. Device according to one or more of the preceding claims, **characterized in that** the frame of the apparatus acquiring images can be moved such that the scanning plane along which an image is acquired is moved in various orientation conditions, the frame supporting the needle or the guide cannula being fixed to said movable frame of the apparatus so that the needle or cannula are moved in space in a way corresponding to the movement of the scanning plane.

15. Device according to one or more of the preceding claims, **characterized in that** the frame of the apparatus acquiring images can be angularly moved about an axis parallel or included in the scanning plane, said scanning plane being angularly rotated with respect to said axis and the needle or guide cannula being also rotated together with said scanning plane and at the same angular extent, so that the axis of the needle or neddle-guide cannula maintain the parallel relation or said axis remains included inside the scanning plane for any orientation thereof

16. Device according to one or more of the preceding claims, **characterized in that** the needle or needle-guide cannula are supported in an adjustable way according to any directions in the scanning plane or in a plane parallel to said scanning plane.

17. Device according to one or more of the preceding claims, **characterized in that** there is provided a curved sliding guide, preferably a circular one and integral with the frame of the apparatus for acquiring images and along which a saddle or slide bearing means supporting the needle and/or guide cannula is mounted that can be moved and fixed in place manually or by powered means.

18. Device according to one or more of the preceding claims, **characterized in that** it provides in combination an apparatus for acquiring diagnostic images, of radiological type, having means for scanning the image along a scanning plane housed in an annular frame, whose inner space is the space for housing the patient the image scanning plane being composed of the median plane subtended by said annular frame, while said annular frame can be tilted about a diametral axis means supporting the needle or the needle guide cannula being composed of an arm fastened to an end of said annular frame and which opposite end extends in the area of the scanning plane and it bears a needle-holder head wherein the needle or cannula are retained in a position coinciding with said scanning plane or with a plane parallel thereto and while the annular frame has a coaxial or substantially coaxial guide that is an annular one or like an annular sector for a saddle or slide moving means fastening the end of the arm supporting the needle.

19. Device according to one or more of the preceding claims, **characterized in that** the needle-holder head is made like a four bar polygon or better like a four bar linkage, wherein the needle or the needle-guide cannula are retained in a position coinciding with a diagonal of said four bar polygon, such that the activation of the four bar linkage determines the to and fro movement for a predetermined travel of the needle or needle-guide cannula in a direction parallel to itself and its longitudinal axis.

20. Device according to one or more of the preceding claims, **characterized in that** the needle-holder head is made like a pantograph, the needle or cannula being borne by a member provided at a pivot point between two links or arms of said pantograph and the pantograph being made such that said pivot point moves along a straight path when opening and closing the pantograph.

21. Device according to one or more of the preceding claims, **characterized in that** the frame supporting the needle and/or cannula is fastened to the frame of the apparatus for acquiring images said supporting frame being movable with respect to said frame of the apparatus for acquiring images only as regards a rotation about an axis perpendicular or transverse to the image acquiring or scanning plane.

22. Device accordino to claim 21, **characterized in that** the frame supporting the needle and/or cannula is fastened to the frame of the apparatus for acquiring images said supporting frame being movable with respect to said frame of the apparatus for acquiring images also in a radial direction with respect to an axis perpendicular or transverse to the image acquiring or scanning plane.

23. Device according to claims 21 or 22, **characterized in that** the frame supporting the needle and/or cannula is fastened to the frame of the apparatus for acquiring images said supporting frame being movable with respect to said frame of the apparatus for acquiring images also in tilting direction of the axis of the needle or cannula wherein said axis ha san orientation intersecting the image acquiring or scanning plane.

24. Device according to one or more of claims 21 to 23, **characterized in that** the frame supporting the needle and/or cannula is fastened to the frame of the apparatus for acquiring images said supporting frame being movable with respect to said frame of the apparatus for acquiring images also in moving direction of the axis of the needle or cannula in a position laterally offset or outside the image acquiring or scanning plane.

25. Device according to one or more of the preceding claims, **characterized in that** to the same apparatus for acquiring images two or more frames supporting the same number of needles or cannulas are fastened.

26. Device according to one or more of the preceding claims, **characterized in that** said frame/frames supporting needles or cannulas are fastened to the frame of the apparatus acquiring images in a movable way or can be disassembled.
